# FASCICULE DE BREVET EUROPEEN

(11) **EP 2 107 906 B1**
(45) Date de publication et mention de la délivrance du brevet: **23.11.2011**
(21) Numéro de dépôt: 07872446.5
(22) Date de dépôt: 27.12.2007
(51) Int. Cl.: A61K 31/519, A61K 31/704, A61K 31/7068, A61K 45/06, A61P 35/02

(54) **NOUVELLE UTILISATION THERAPEUTIQUE POUR LE TRAITEMENT DES LEUCEMIES**
VERWENDUNG EINES NEUEN THERAPEUTIKUMS ZUR BEHANDLUNG VON LEUKÄMIE
NOVEL THERAPEUTIC USE FOR TREATING LEUKAEMIA

(30) Priorité: 28.12.2006 FR 0611492
(43) Date de publication de la demande: 14.10.2009
(73) Titulaire: SANOFI, 75013 Paris (FR)
(72) Inventeur: BOURRIE, Bernard, F-75013 Paris (FR); CASELLAS, Pierre, F-75013 Paris (FR)
(74) Mandataire: Lavé, Stéphanie
(86) Numéro de dépôt international: PCT/FR2007/002171
(87) Numéro de publication internationale: WO 2008/102075

(56) Documents cités:
- WO-A-2006/016067
- WO-A-2007/003765
- MANLEY ET AL: "Advances in the structural biology, design and clinical development of Bcr-Abl kinase inhibitors for the treatment of chronic myeloid leukaemia" BIOCHIMICA ET BIOPHYSICA ACTA (BBA) - PROTEINS & PROTEOMICS, ELSEVIER, vol. 1754, no. 1-2, 30 décembre 2005 (2005-12-30), pages 3-13, XP005214189 ISSN: 1570-9639
- SCHROEDER M C ET AL: "SOLUBLE 2-SUBSTITUTED AMINOPYRIDOÄ2,3-DÜPYRIMIDIN-7-YL UREAS. STRUCTURE-ACTIVITY RELATIONSHIPS AGAINST SELECTED TYROSINE KINASES AND EXPLORATION OF IN VITRO AND IN VIVO ANTICANCER ACTIVITY" JOURNAL OF MEDICINAL CHEMISTRY, AMERICAN CHEMICAL SOCIETY. WASHINGTON, US, vol. 44, no. 12, 7 juin 2001 (2001-06-07), pages 1915-1926, XP001152609 ISSN: 0022-2623

## Description

Cette invention concerne le traitement des leucémies, notamment des leucémies myéloïdes comme défini dans la revendication 1.

La leucémie est une maladie cancéreuse de la moelle osseuse et du sang. On peut distinguer quatre types de leucémie : leucémie myéloïde chronique, leucémie myéloïde aigue, leucémie lymphoïde chronique, leucémie lymphoïde aigue.

Les leucémies myéloïdes de type aigu, avec une progression rapide sont nommées AML ou Leucémie Myéloïde Aigue. Les leucémies myéloïdes de type chronique avec une progression graduelle moins agressive sont nommées CML ou Leucémie Myéloïde Chronique. Il s'agit de maladies clonales de la moelle osseuse caractérisée par une expansion clonale des cellules myéloïdes qui ne peuvent se différencier normalement et s'accumulent dans la moelle osseuse et le sang.

En 2006, aux Etats-Unis, selon une étude de l'American Cancer Society, on estime qu'il sera diagnostiqué 11930 nouveaux cas d'AML et 4500 nouveaux cas de CML. Sur la période de 2002 à 2006, le taux de survie à 5 ans est de 20,4 % pour l'AML et de 42,3% pour la CML (Cancer and Facts Figures 2006, American Cancer Society, www.leukemia-lymphoma.org/).

Selon la classification French-American-British (FAB) de 1976, il existe 8 sous-types d'AML, désignés M0 à M7, selon le type de cellules à partir desquelles la leucémie se développe (Bennett et al, 1976, "Proposais for the classification of the acute leukaemias. French-American-British (FAB) co-operative group". Br J Haematol 33 (4): 451-8).

Environ 95% des patients atteints de CML portent une translocation génétique entre les chromosomes 9 et 22 des cellules leucémiques. Cette anomalie connue en tant que chromosome Philadelphia (Ph1) provoque une prolifération et une multiplication incontrôlée de tous les types de globules blancs et des plaquettes.

Actuellement, plusieurs médicaments pour le traitement des leucémies sont disponibles. Cependant, il demeure un besoin de nouveaux composés thérapeutiques actifs pour l'amélioration des stratégies de traitement des patients atteints de leucémie ou le développement de traitement alternatif aux traitements déjà connus (Plo et al, Mol Pharmacol, 2002, 62 :304-312).

Le produit N-[2-(2,1,3-benzothiadiazol-5-ylamino)-6-(2,6-dichlorophényl) pyrido[2,3-d]pyrimidin-7-yl]-N'-(1,1-diméthyléthyl)-urée est décrit dans la demande internationale WO2007/003765. Sa formule est présentée ci-dessous :

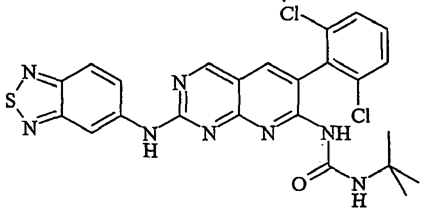

Un procédé de préparation du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée est également décrit.

Bien que ce composé puisse présenter une activité anti-tumorale significative sur des cellules par des tests in vitro, de nouveaux paramètres, tels que la distribution du composé dans les tissus, la quantité de produit dans le sérum, la pharmacocinétique, et le métabolisme participent à l'obtention d'un effet in vivo, non prédictible à partir de tests in vitro. Il a d'ailleurs été démontré qu'une activité anti-tumorale in vitro n'est pas toujours prédictive d'une activité in vivo (Cancer Res. 1988 Oct 1;48(19):5447-54, Cancer Chemother Pharmacol. 1996 38 :548-552).

Par des tests in vivo, on a démontré que le composé N-[2-(2,1,3-benzothiadiazol-5-ylamino)-6-(2,6-dichlorophényl)pyrido[2,3-d]pyrimidin-7-yl]-N'-(1,1-diméthyléthylrurée présente une activité in vivo anti-tumorale significative sur des animaux porteurs de leucémies humaines.

L'objet de la présente invention est l'utilisation du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée ou un hydrate, un sel ou un solvate de celui-ci, pour la préparation d'un médicament destiné au traitement des leucémies comme défini dans la revendication 1. Par leucémie, on entend des leucémies telles que la leucémie myéloïde chronique, la leucémie myéloïde aigue, la leucémie lymphoïde chronique, la leucémie lymphoïde aigue, et les divers syndromes myéloprolifératifs.

En particulier, la présente invention concerne l'utilisation du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée, ou un hydrate, un sel ou un solvate de celui-ci, pour la préparation d'un médicament destiné au traitement des leucémies myéloïdes. Plus particulièrement, la présente invention concerne l'utilisation du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée, ou un hydrate, un sel ou un solvate de celui-ci, pour la préparation d'un médicament destiné au traitement des leucémies de type AML. Plus particulièrement, la présente invention concerne l'utilisation du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée, ou un hydrate, un sel ou un solvate de celui-ci, pour la préparation d'un médicament destiné au traitement des leucémies de type CML.

L'objet de la présente invention se rapporte aux utilisations citées plus haut pour le traitement des mammifères, en particulier de l'homme.

Dans la présente invention, le composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée peut être administré chez l'animal testé par voie intra-péritonéale, ou encore par voie intraveineuse suivie d'une voie intra-péritonéale, ou encore par voie intraveineuse suivie d'une voie orale.

Dans la présente invention, le composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée est typiquement formulé pour une administration sous la forme d'une composition acceptable au plan pharmaceutique. Ces compositions pharmaceutiques contiennent une dose efficace du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée ou un sel pharmaceutiquement acceptable, un hydrate ou solvate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale ou intraveineuse, le composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

On utilise de préférence une formulation PEG400 22% / Solutol 5% / G5 73% pour le traitement par voie intraveineuse des souris portant des tumeurs Kasumi1.

On utilise de préférence une formulation Labrasol 21% / Solutol 5% / HCl 0.001 N 74% pour le traitement par voie orale des souris portant des tumeurs Kasumi1 ou KG1.

On utilise de préférence une formulation PEG400 22% / Solutol 5% / G5 73% pour le traitement par voie intra-péritonéale des souris portant des tumeurs EOL-1.

On utilise de préférence une formulation PEG400 22% / Solutol 5% / G5 73% pour le traitement, par voie intraveineuse suivie d'une voie intra-péritonéale, des souris portant des tumeurs CTV1.

On utilise de préférence une formulation DMSO 5% / Tween80 10% / H₂O 85% pour le traitement, par voie orale, ou par voie intraveineuse suivie d'une voie intra-péritonéale, ou encore par voie intraveineuse suivie d'une voie orale, des souris portant des tumeurs KG1a.

On utilise de préférence une formulation DMSO 5% / Tween80 10% / H₂O 85% pour le traitement, par voie intraveineuse suivie d'une voie intra-péritonéale, des souris portant des tumeurs K562 ou CMLT1.

On utilise de préférence une formulation DMSO 5% / Tween80 10% / H₂O 85% pour le traitement, par voie intraveineuse, des souris portant des tumeurs KG1.

Les formes unitaires d'administration appropriées comprennent les formes par voie orale telles que les comprimés, les gélules molles ou dures, les poudres, les granules et les solutions ou suspensions orales et les formes d'administration intraveineuse.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La thérapeutique par le composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée selon la présente invention peut être utilisée en même temps que d'autres thérapeutiques. Notamment, le composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée selon l'invention peut être administré en association avec un (ou plusieurs) principe(s) actif(s) anticancéreux, en particulier des composés antitumoraux tels que
les agents alkylants tels que les alkylsulfonates (busulfan), la dacarbazine, la procarbazine, cloretazine, les moutardes azotées (chlorméthine, melphalan, chlorambucil, cyclophosphamide, ifosfamide), les nitrosourées tels que la carmustine, la lomustine, la sémustine, la streptozocine faltretamine;
les alcaloïdes antinéoplasiques tels que la vincristine la vinblastine, la vinorelbine, la vindesine;
les taxanes tel que le paclitaxel ou le taxotère ;
les antibiotiques antinéoplasiques tels que l'actinomycine, la bleomycine;
les agents intercalants tels la mitoxantrone;
les antimétabolites antinéoplasiques: les antagonistes des folates, le méthotrexate; les inhibiteurs de la synthèse des purines; les analogues de la purine tels que mercaptopurine, 6-thioguanine; les inhibiteurs de la synthèse des pyrimidines, les inhibiteurs d'aromatase, la capécitabine, les analogues de la pyrimidine tels que fluorouracil, gemcitabine, cytarabine et cytosine arabinoside; le bréquinar, la nelarabine;
les inhibiteurs de topoisomérases tels que l'irinotecan, l'exatecan, le topotecan, le teniposide, la camptothécine ou l'étoposide;
les agonistes et antagonistes hormonaux anticancéreux incluant le tamoxifène;
les inhibiteurs de kinase, tels que l'imatinib, le nilotinib et le dasatinib, la midaustorin, le sorafenib, le lestaurtinib, le tandutinib;
les inhibiteurs de facteurs de croissance;
les antiinflammatoires tels que le pentosane polysulfate, les corticostéroïdes, la prednisone, la dexamethasone;
le ceplene (dichlorhydrate d'histamine);
les anthracyclines tesl que la daunorubicine, l'epirubicine, la pirarubicine, l'idarubicine, la zorubicine, l'aclarubicine, l'annamycine, la doxorubicine, la mitomycine et la méthramycine;
les complexes métalliques anticancéreux, les complexes du platine, le cisplatine, le carboplatine, l'oxaliplatine, le satraplatine;
l'interféron alpha,
le triphénylthiophosphoramide;
les agents antiangiogéniques;
la thalidomide;
les inhibiteurs de la farnesyl-tranferase tel le tipifarnib;
les inhibiteurs de l'ADN methyltransferase tel le MG98 ;
les adjuvants d'immunothérapie tel le gemtuzumab ozogamicin, le HuM 195;
les agents biothérapeutiques tel le CT388-IL3;
les antisens tel le GTI-2040 ;
les vaccins.

Plus particulièrement, le composé N-[2-(2,1,3-benzothiadiazo)-5-ylamino)-6-(2,6-dichlorophényl)pyrido[2,3-d]pyrimidin-7-yl]-N'-(1,1-diméthyléthyl)-urée selon l'invention peut être administré en association avec un ou plusieurs composé(s) de la famille des anthracyclines.

Plus particulièrement, le composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée selon l'invention peut être administré en association avec la daunorubicine ou en association avec la cytosine arabinoside, ou bien en association avec la daunorubicine et la cytosine arabinoside.

Selon la présente invention, le composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée peut également être administré en association avec un ou plusieurs autres principes actifs utiles dans une des pathologies indiquées ci-dessus, par exemple un agent anti-émétique, anti-douleur, anti-inflammatoire, anti-cachexie.

Il est également possible d'associer aux composés de la présente invention un traitement par des radiations.

Ces traitements peuvent être administrés simultanément, séparément, séquentiellement et/ou espacés dans le temps. Le traitement sera adapté par le praticien en fonction du malade à traiter.

Dans la présente invention, le produit N-[2-(2,1,3-benzothiadiazol-5-ylamino)-6-(2,6-dichlorophényl)pyrido[2,3-d]pyrimidin-7-yl]-N'-(1,1-diméthyléthyl)-urée est administré selon une posologie qui permet le traitement des leucémies. La posologie varie selon la voie d'administration et selon les caractéristiques physiques du patient. Les posologies convenant à cette fin comprennent celles qui présentent une efficacité thérapeutique pour le traitement des troubles résultant d'une prolifération cellulaire anormale. Le produit N-[2-(2,1,3-benzothiadiazol-5-ylamino)-6-(2,6-dichlorophényl)pyrido[2,3-d]pyrimidin-7-yl]-N'-(1,1-diméthyléthyl)-urée peut être administré aussi souvent qu'il est nécessaire pour obtenir l'effet thérapeutique recherché.

L'efficacité du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée sur les leucémies peut être déterminée expérimentalement comme dans l'exemple suivant qui illustre l'invention.

### EXEMPLE

### Matériaux et méthodes

On utilise des souris Scid CB-17 (fournies par Charles River, Lyon, France). Lors de la randomisation, les animaux ont un poids moyens de 20-22 g et sont âgés de 6 à 9 semaines.

Les animaux sont reçus au moins un mois avant l'expérience pour permettre une parfaite acclimatation. La santé des animaux est examinée le jour qui précède l'implantation de la tumeur et avant la randomisation afin de s'assurer que seuls des animaux en bonne santé seront utilisés pour l'expérimentation. Ils sont placés dans des cages macrolon type III avec des capots filtrants (au maximun 8 souris par cage) dans une pièce stérile où l'air est continuellement filtré pour éviter toute contamination. La stérilité de la pièce est vérifiée une fois par mois. Les cages sont stérilisées à 121°C avant utilisation et changées deux fois par semaine. La température de la pièce est maintenue à 22°C et l'humidité relative à 60+/-10%. Les animaux sont placés en condition d'un cycle naturel de lumière. L'eau est stérilisée à 121°C pendant 30 minutes. La consommation d'eau est suivie visuellement chaque jour, et les bouteilles sont changées 2 fois par semaine. La nourriture et l'eau sont données *ad libitum.* La litière est stérilisée à 121°C pendant 30 minutes et changée deux fois par semaine.

Le jour qui précède la première administration d'un composé, les animaux portant des tumeurs, sont classés en plusieurs groupes. Seuls les animaux porteurs de deux tumeurs palpables ou de poids déterminés sont sélectionnés et distribués au hasard dans les groupes traités et les groupes de contrôle. Chaque groupe est constitué de 5 à 10 souris. En début d'étude, chaque cage est étiquetée avec une fiche indiquant le jour d'implantation de la tumeur, le type de tumeur, le composé testé et le mode d'administration.

L'implantation des tumeurs est réalisée comme suit: après retrait de la tumeur de la souris donneuse, la tumeur est découpée en fragments de 2 à 3 mm de diamètre, placée dans un tampon phosphate salin, et implantée bilatéralement avec un trocart adapté.

### Détermination de l'activité anti-tumorale

Le volume des tumeurs et sa conversion en poids est estimé selon la formule: poids (en mg) = (a × b²)/2, où a et b sont respectivement la longueur et la largeur de la tumeur (mm). Les tumeurs sont mesurées deux fois par semaine avec un pied à coulisse. Dans les tableaux suivants, P indique le poids des tumeurs en début de traitement.

Deux paramètres d'estimation de l'activité antitumorale sont utilisés: le log₁₀ de cellules tuées (Log cell kill) et le T/C.
- Calcul du log₁₀ de cellules tuées = (T - C) / 3.32 × Td, où (T - C) est le délai de croissance de la tumeur et Td le temps de doublement du volume (et du poids) de la tumeur (exprimé en jours). T est le temps médian en jours, d'atteinte d'une valeur fixe (ex:1000 mg) dans le groupe Traité, et C est le temps médian en jours, d'atteinte de cette même valeur dans le groupe Contrôle. Une valeur du log₁₀ de cellules tuées > 0.7 est indicative d'une activité antitumorale de la molécule. Une valeur du log₁₀ de cellules tuées > 2.8 est indicative d'une activité antitumorale très élevée de la molécule (J Liang et al, Invest New Drugs 2005;23(3):213-24).
- Calcul du T/C: Les groupes traités et les groupes contrôles sont évalués lorsque les tumeurs du groupe contrôle atteignent approximativement 1000 mg (valeur médiane du groupe). Le poids médian des tumeurs de chaque groupe traité est alors déterminé. La valeur T/C ((poids des tumeurs des groupes Traités / poids des tumeurs des groupes Contrôles) × 100) en pourcentage est une indication de l'efficacité anti-tumorale : une valeur T/C équivalente ou inférieure à 42 % est indicative d'une activité anti-tumorale selon l'Institut National du Cancer Américain (NCI). Une valeur T/C inférieure à 10% est représentative d'une activité anti-tumorale très élevée.

- Le nombre de souris ne présentant plus de tumeurs longtemps après la dernière administration (TFS = tumor-free survival), et considérées comme guéries, peut également constituer un paramètre d'activité de la molécule.
- Evaluation de la toxicité du composé testé: Une perte de poids égale ou supérieure à 20% ou l'apparition d'une létalité quelconque en relation avec le composé est considérée comme un traitement toxique excessif.

A titre d'exemples, sont donnés, dans les tableaux 1 à 7 suivants les résultats obtenus avec le composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée.

**TABLEAU 1**

| *Activité du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée sur des souris portant des tumeurs KG1a (AML)* | | | | | | | |
|---|---|---|---|---|---|---|---|
| Formulation | P mg | Dose mg/kg/injection | Voie | Administration aux jours : | Dose totale mg/kg | T/C à 22 jours | Log₁₀ cellules tuées (TFS) |
| DMSO 5% Tween80 10% H₂O 85 % | 91-98 | 17 | IV/IP | 5-13,15/16, 18-20,22,24-26,29,31 | 340 | 0% | »6 80% au jour 120) |
| DMSO 5% Tween8010% H₂O 85% | 147 | 40 × 2 (2 administrations par jour de traitement) | orale | 15-44 | 2400 | 14,9% | 3.0 |
| DMSO 5% Tween80 10% H₂O 85% | 1000 | 25 / 40 × 2 (2 administrations orales par jour de traitement) | IV/orale | 22-30/31-44 | 225 / 1120 | non pertinent | >>6 |

| | | | | | | | |
|---|---|---|---|---|---|---|---|
| On entend par IV/IP une administration par voie intraveineuse suivie d'une voie intra-péritonéale, ici la transition s'effectue entre le jour 15 et le jour 16. On entend par IV/orale une administration par voie intraveineuse suivie d'une voie orale, ici la transition s'effectue entre le jour 30 et le jour 31. Le traitement par voie IV des jours 22 à 30 à la dose de 25mg/kg de tumeurs au stade très avancées (1000mg), conduit à une réduction de la masse tumorale de 80%. Au jour 31, le composé est administré par voie orale jusqu'au jour 44. A la fin de cette deuxième période de traitement, les tumeurs ne sont plus mesurables (<63mg). La lignée cellulaire KG-1a (AML) est décrite par Koeffler et al., Blood 56: 265 (1980), et délivrée par DSMZ n°ACC 421, Deutsche Sammlung von Mikroorganismen und Zellkulturen GmbH) Mascheroder Weg 1 b, 38124 Braunschweig, Germany). | | | | | | | |

| *Activité du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée sur dessouris portant des tumeurs KG 1 (AML)* | | | | | | | |
|---|---|---|---|---|---|---|---|
| Formulation | P mg | Dose mg/kg/injection | Voie | Administration aux jours : | Dose totale mg/kg | T/C à 28 jours | Log₁₀ cellules tuées (TFS) |
| DMSO 5% Tween80 10% H₂O 85 % | 127-130 | 17 | IV | 19-28, 30, 32, 34, 36, 38 | 255 | 0% | >6 (100%) |
| Labrasol 21% Solutol 5% HCl 0.001 N 74% | 130-132 | 40 × 2 (2 administrations par jour de traitement) | Orale | 18-24, 26, 28, 30, 32, 34, 36, 38, 40 | 1200 | 0% | 4.3 |

| | | | | | | | |
|---|---|---|---|---|---|---|---|
| On entend par IV une administration par voie intraveineuse. La lignée cellulaire KG-1 (AML) est décrite par Koeffler et al., Science 200: 1153-1154 (1978), et délivrée par DSMZ n°ACC 14. | | | | | | | |

**TABLEAU 3**

| *Activité du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée sur des souris portant des tumeurs Kasumi1(AML)* | | | | | | | |
|---|---|---|---|---|---|---|---|
| Formulation | P mg | Dose mglkg/injection | Voie | Administration aux jours : | Dose totale mg/kg | T/C à 28 jours | Log₁₀ cellules tuées |
| PEG400 22% Solutol 5% G5 73% | 178 | 15 × 2 (2 administrations par jour de traitement) | IV | 25-31;33;34. (Une seule administration au jour 30) | 255 | 20% | 3 |
| Labrasol 21% Solutol 5% HCl 0.001 N 74% | 178 | 40 × 2 (2 administrations par jour de traitement) | orale | 25-31;33;34. (Une seule administration au jour 30) | 680 | 17,5% | 2,4 |

| | | | | | | | |
|---|---|---|---|---|---|---|---|
| On entend par IV une administration par voie intraveineuse. La lignée cellulaire Kasumi-1 (AML) est décrite par Asou et al., Blood 77: 2031 (1991), et délivrée par DSMZ n°ACC 220. | | | | | | | |

**TABLEAU 4**

| *Activité du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée sur des souris portant des tumeurs EOL-1 (AML)* | | | | | | | |
|---|---|---|---|---|---|---|---|
| Formulation | P mg | Dose mg/kg/injection | Voie | Administration aux jours : | Dose totale mg/kg | T/C à 14 jours | Log₁₀ cellules tuées |
| PEG400 22% Solutol 5% G5 73% | 133-146 | 15 x 2 (2 administrations par jour de traitement) | IP | 8-10; 12-17; 19 | 300 | 5.7% | 3.1 |

| | | | | | | | |
|---|---|---|---|---|---|---|---|
| On entend par IP une administration par voie intra-péritonéale. La lignée cellulaire EOL-1 (AML) est décrite par Saito et al., Blood 66: 1233-1240 (1985), et délivrée par DSMZ n°ACC 386. | | | | | | | |

**TABLEAU 5**

| *Activité du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée sur des souris portant des tumeurs CTV1 (AML)* | | | | | | | |
|---|---|---|---|---|---|---|---|
| Formulation | P mg | Dose mg/kg/injection | Voie | Administration aux jours : | Dose totale mg/kg | T/C à 25 jours | Log₁₀ cellules tuées |
| PEG400 22% Solutol 5% G573% | 100 | 25 | IV/IP | 17-23; 25-26 / 28-32 | 350 | 1.6% | 1.7 |

| | | | | | | | |
|---|---|---|---|---|---|---|---|
| On entend par IV/IP une administration par voie intraveineuse suivie d'une voie intra-péritonéale, ici la transition s'effectue entre le jour 26 et le jour 28. La lignée cellulaire CTV1 (AML) est décrite par Chen et al., Gann 75: 660-664 (1984), et délivrée par DSMZ n°ACC 40. | | | | | | | |

**TABLEAU 6**

| *Activité du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée sur des souris portant des tumeurs K562 (CML)* | | | | | | | |
|---|---|---|---|---|---|---|---|
| Formulation | P mg | Dose mg/kg/injection | Voie | Administration aux jours : | Dose totale mg/kg | T/C à 28 jours | Log₁₀ cellules tuées (TFS) |
| DMSO 5% Tween80 10% H₂O 85 % | 63-80 | 25 | IV/IP | 4-11/12-25 | 550 | 0% | 4,2 (43% au jour 130) |

| | | | | | | | |
|---|---|---|---|---|---|---|---|
| On entend par IV/IP une administration par voie intraveineuse suivie d'une voie intra-péritonéale, ici la transition s'effectue entre le jour 11 et le jour 12. La lignée cellulaire K-562 (CML) est décrite par Lozzio et al., J Natl Cancer Inst 50: 535 (1973), par Lozzio et al., Blood 45: 321 (1975), et délivrée par DSMZ n°ACC 10. | | | | | | | |

**TABLEAU 7**

| *Activité du composé N-[2-(2,1,3-benzothiadiazol-5-ylamino)-6-(2,6-dichlorophényl)pyrido[2,3-d]pyrimidin-7-yl]-N'-(1,1-diméthyléthyl)-urée sur des souris portant des tumeurs CMLT1 (CML)* | | | | | | | |
|---|---|---|---|---|---|---|---|
| Formulation | P mg | Dose mg/kg/injection | Voie | Administration aux jours : | Dose totale mg/kg | T/C à 22 jours | Log₁₀ cellules tuées (TFS) |
| DMSO 5% Tween80 10% H₂O 85 % | palpable ~30 | 20 | IV/IP | 3-7/10-14; 17-21 | 300 | 1% | 1.5 (50% au jour 42) |

| | | | | | | | |
|---|---|---|---|---|---|---|---|
| On entend par IV/IP une administration par voie intraveineuse suivie d'une voie intra-péritonéale, ici la transition s'effectue entre le jour 7 et le jour 10. La lignée cellulaire CMLT1 (CML) est décrite par Kuriyama et al. dans Blood, 74: 1989, 1381-1387, par Soda et al. dans British Journal of Haematology, 59: 1985, 671-679 et par Drexler, dans Leukemia Research, 18: 1994, 919-927 et délivrée par la société DSMZ n°ACC 7. | | | | | | | |

## Revendications

1. Composé N-[2-(2,1,3-benzothiadiazol-5-ylamino)-6-(2,6-dichlorophényl) pyrido[2,3-d]pyrimidin-7-yl]-N'-(1,1-diméthyléthyl)-urée ou un hydrate, un sel ou un solvate de celui-ci, pour son utilisation pour le traitement des leucémies myéloïdes, pour une administration par voie intra-péritonéale, par voie intraveineuse suivie d'une administration par voie intra-péritonéale ou par voie intraveineuse suivie d'une administration par voie orale.

2. Composé pour son utilisation selon la revendication 1, pour le traitement des leucémies de type AML.

3. Composé pour son utilisation selon la revendication 2, pour une administration par voie intra-péritonéale.

4. Composé pour son utilisation selon la revendication 2, pour une administration par voie intraveineuse, suivie d'une administration par voie intra-péritonéale.

5. Composé pour son utilisation selon la revendication 2, pour une administration par voie intraveineuse, suivie d'une administration par voie orale.

6. Composé pour son utilisation selon la revendication 1, pour le traitement des leucémies de type CML.

7. Composé pour son utilisation selon la revendication 6, pour une administration par voie intra-péritonéale.

8. Composé pour son utilisation selon la revendication 6, pour une administration par voie intraveineuse, suivie d'une administration par voie intra-péritonéale.

9. Composé pour son utilisation selon la revendication 6, pour une administration par voie intraveineuse, suivie d'une administration par voie orale.

10. Composé pour son utilisation selon l'une quelconque des revendications 1 à 9, **caractérisé en ce qu'**il est administré en association avec un ou plusieurs composé(s) de la famille des anthracyclines.

11. Composé pour son utilisation selon l'une quelconque des revendications 1 à 9, **caractérisé en ce qu'**il est administré en association avec la daunorubicine ou en association avec la cytosine arabinoside, ou bien, en association avec la daunorubicine et la cytosine arabinoside.

## Claims

1. Compound N-[2-(2,1,3-benzathiadiazol-5-ylamino)-6-(2,6-dichlorophenyl)pyrido[2,3-d]pyridmidin-7-yl]-N'-(1,1-dimethylethyl) urea or a hydrate, a salt or a solvate thereof, for its use for treating myeloid leukaemias, for administration by the intraperitoneal route, by the intravenous route followed by administration by the intraperitoneal route or by the intravenous route followed by administration by the oral route.

2. Compound for its use according to Claim 1, for the treatment of leukaemias of the AML type.

3. Compound for its use according to Claim 2, for administration by the intraperitoneal route.

4. Compound for its use according to Claim 2, for administration by the intravenous route, followed by administration by the intraperitoneal route.

5. Compound for its use according to Claim 2, for administration by the intravenous route, followed by administration by the oral route.

6. Compound for its use according to Claim 1, for the treatment of leukaemias of CML type.

7. Compound for its use according to Claim 6, for administration by the intraperitoneal route.

8. Compound for its use according to Claim 6, for administration by the intravenous route, followed by administration by the intraperitoneal route.

9. Compound for its use according to Claim 6, for administration by the intravenous route, followed by administration by the oral route.

10. Compound for its use according to any one of Claims 1 to 9, **characterized in that** it is administered in combination with one or more compound (s) of the anthracycline family.

11. Compound for its use according to any one of Claims 1 to 9, **characterized in that** it is administered in combination with daunorubicin or in combination with cytosine arabinoside, or else in combination with daunorubicin and cytosine arabinoside.

## Patentansprüche

1. Die Verbindung N-[2-(2,1,3-Benzothiadiazol-5-ylamino)-6-(2,6-dichlorphenyl)pyrido[2,3-d]pyrimidin-7-y1]-N'-(1,1-dimethylethyl)harnstoff oder ein Hydrat, Salz oder Solvat davon, für ihre/seine Verwendung zur Behandlung von granulozytären Leukämien, für eine Verabreichung über den intraperitonealen Weg, über den intravenösen Weg mit anschließender Verabreichung über den intraperitonealen Weg oder über den intravenösen Weg mit anschließender Verabreichung über den oralen Weg.

2. Zusammenset:zung zur Verwendung nach Anspruch 1 für die Behandlung von Leukämien des AML-Typs.

3. Zusammensetzung zur Verwendung nach Anspruch 2 für eine Verabreichung über den intraperitonealen Weg.

4. Zusammensetzung zur Verwendung nach Anspruch 2 für eine Verabreichung über den intravenösen Weg mit anschließender Verabreichung über den intraperitonealen Weg.

5. Zusammensetzung zur Verwendung nach Anspruch 2 für eine Verabreichung über den intravenöser. Weg mit anschließender Verabreichung über den oralen Weg.

6. Zusammensetzung zur Verwendung nach Anspruch 1 für die Behandlung von Leukämien des CML-Typs.

7. Zusammensetzung zur Verwendung nach Anspruch 6 für eine Verabreichung über den intraperitonealen Weg.

8. Zusammensetzung zur Verwendung nach Anspruch 6 für eine Verabreichung über den intravenösen Weg mit anschließende Verabreichung über den intraperitonealen Weg.

9. Zusammensetzung zur Verwendung nach Anspruch 6 für eine Verabreichung über den intravenösen Weg mit anschließende Verabreichung über den oralen Weg.

10. Zusammensetzung zur Verwendung nach einem der Ansprüche 1 bis 9, **dadurch gekennzeichnet, dass** sie in Kombination mit einer oder mehreren Verbindung(en) der Anthracyclin-Familie verabreicht wird.

11. Zusammensetzung zur Verwendung nach einem der Ansprüche 1 bis 9, **dadurch gekennzeichnet, dass** sie in Kombination mit Daunorubicin oder in Kombination mit Cytosinarabinosid oder in Kombination mit Daunorubicin und Cytosinarabinosid verabreicht wirt.
